# EUROPEAN PATENT SPECIFICATION

(11) **EP 2 415 452 B1**
(45) Date of publication and mention of the grant of the patent: **02.11.2016**
(21) Application number: 10758227.2
(22) Date of filing: 26.03.2010
(51) Int. Cl.: A61K 8/46, A61K 8/36, A61K 8/39, A61Q 5/02, C11D 1/29, C11D 1/72, C11D 3/20, C11D 3/48, C11D 17/08

(54) **AQUEOUS HAIR CLEANSING AGENT**
WÄSSRIGES HAARWASCHMITTEL
AGENT AQUEUX DE NETTOYAGE DES CHEVEUX

(30) Priority: 31.03.2009 JP 2009084332; 31.03.2009 JP 2009084335
(43) Date of publication of application: 08.02.2012
(73) Proprietor: Kao Corporation, Chuo-Ku Tokyo 103-8210 (JP)
(72) Inventor: TERADA, Eiji, Tokyo 131-8501 (JP)
(74) Representative: Hoffmann Eitle
(86) International application number: PCT/JP2010/002179
(87) International publication number: WO 2010/113447

(56) References cited:
- EP-A1- 0 453 238
- JP-A- 7 187 946
- JP-A- 49 099 104
- JP-A- 2006 347 918
- JP-A- 2007 131 583
- JP-A- 2007 254 355
- JP-A- 2007 254 356
- JP-A- 2008 063 294

## Description

### TECHNICAL FIELD

The present invention relates to an aqueous hair cleansing agent.

### BACKGROUND ART

A principal purpose of a hair cleansing agent resides in removing dirt on hair and scalp, and keeping the hair and scalp clean. In view of obtaining a hair cleansing agent having a high cleansing performance, it is preferable to use, as a main cleansing component, an anionic surfactant charged at the equivalent level with the hair and scalp. It is more preferable to use a strong-acid-based anionic surfactant having a sulfuric acid group or sulfonic acid group in the anionic portion thereof, in view of preventing re-adsorption of dirt. Among others, an alkyl sulfate and alkyl ether sulfate are most generally used as the main component of a hair cleansing agent, by virtue of their rich foaming property and excellent cleansing performance.

The alkylsulfate and alkyl ether sulfate, however, still have problems in terms of mildness to the scalp. Horny layer cells which form 10 to 20 layers in the surficial portion of the skin has important roles of keeping moisture within the skin, and protection from an external stimulation. The above-described alkyl sulfate and alkyl ether sulfate, however, permeate into the horny layer, strongly swell the cells, and elutes natural moisturizing components, such as an amino acid or lipid inherent to the cells, out from the cells. Accordingly, repetitive use of such hair cleansing agent, containing these surfactants as the main component, may tend to dry the skin due to the lowered moisture content of the scalp, and to induce itchiness of the scalp because the surfactant and some external stimulating substance may more readily permeate into the skin.

A known strategy for adding mildness to the scalp, typically by suppressing swelling of the horny layer, relates to a method of formulating a hair cleansing agent using an amino acid-based anionic surfactant as the main component, in place of the alkyl ether sulfate. The amino acid-based surfactant is generally believed to be less likely to swell the horny layer, and is therefore mild to the skin. The anionic portion thereof is, however, often weakly acidic such as carboxylic acid, so that the hair cleansing agent has been limited in the foamability and cleansing performance, only to prove that it has been insufficient to fulfill the basic performances as a hair cleansing agent. Aiming at solving the problem, a cleansing composition proposed in Patent Document 1 uses, as the main component, an anionic surfactant in the form of alkyloylalkyl taurine salt, having a sulfonic acid group in the anionic portion thereof. The composition, however, still has limited foamability and richness of foam as compared with the alkylsulfate and alkyl ether sulfate, and it has remained difficult to fulfill the basic performance requirements of a hair cleansing agent.

Another challenge to achieve both of mildness and foamability may be found in a cleansing agent composition proposed in Patent Document 2, characterized by using an anionic surfactant such as alkyl ether sulfate, in combination with a nonionic surfactant such as polyethoxylated alcohol, which has an alkyl group having 8 to 16 carbon atoms in the alcohol portion thereof, having 10 to 45 ethoxy groups per molecule, and having a pH value of 6.0 to 8.0. A similar challenge may be found in a shampoo composition proposed in Patent Document 3, characterized by using an anionic surfactant such as alkyl ether sulfate, in combination with a nonionic surfactant having an HLB value of at least 8.

The nonionic surfactant is generally known to show small swellability of the horny layer, and to be mild to the skin, but is difficult to obtain rich foam favored for a hair cleansing agent. The nonionic surfactant is used in an amount of at least 38% by weight in the composition according to the above-described Patent Document 2, and in an amount of at least 29% by weight in the composition according to Patent Document 3. Use of such relatively large amounts of nonionic surfactants has, however, failed in obtaining rich foam favored for a hair cleansing agent, and has prevented mildness and foamability from being attained at the same time.

### RELATED DOCUMENT

### [PATENT DOCUMENT]

[Patent Document 1] Japanese Patent Publication No. JP-AS61-272295
[Patent Document 2] Japanese Patent Publication No. JP-AS49-099104
[Patent Document 3] Japanese Patent Publication No. JP-AH04-225909
[Patent Document 4] Japanese Patent Publication No. JP-A-2001-131592

### SUMMARY OF THE INVENTION

### [Problems to be Solved by the Invention]

The present invention provides an aqueous hair cleansing agent which is excellent in foamability, smoothness of foam, finger combability during rinsing, mildness to the scalp, and less causative of itchiness.

### [Means for Solving the Problems]

The present inventor have found that swelling of the horny layer may be suppressed, and mildness to the scalp and excellent foamability may be attainable at the same time, with respect to an aqueous hair cleansing agent having an alkylsulfate or alkyl ether sulfate contained as a main cleansing component, by mixing a small amount of polyoxyethylene alkyl ether-type nonionic surfactant having an alkyl group of a specific chain length.

According to the present invention, there is provided an aqueous hair cleansing agent which includes the following components (A), (B):
(A) a sulfate-type anionic surfactant represented by the following general formula (1):

   R¹O(CH₂CH₂O)ₙSO₃M (1)

   wherein, in the general formula (1), R¹ represents an alkyl group or alkenyl group having 10 to 18 carbon atoms, M represents a cation originated from an alkali metal, alkali earth metal, ammonium, alkanolamine or basic amino acid, and n represents a number of 0 to 5 estimated based on weight average; and
(B) polyoxyethylene alkyl ether-type nonionic surfactant represented by the following general formula (2):

   R²O(CH₂CH₂O)ₘH (2)

   wherein, in the general formula (2), R² represents a straight-chain or branched alkyl group or alkenyl group having 18 carbon atoms, and m represents n number of 6 to 10 estimated based on weight average,
   and further comprising water,
   wherein the ratio of the component (B), relative to the total of the components (A) and (B) is from 1 to 25% based on the weight ratio.

### [Advantageous Effect of the Invention]

The aqueous hair cleansing agent of the present invention is excellent in foamability, smoothness of foam, finger combability during rinsing, mildness to the scalp, and less causative of itchiness.

### BRIEF DESCRIPTION OF THE DRAWINGS

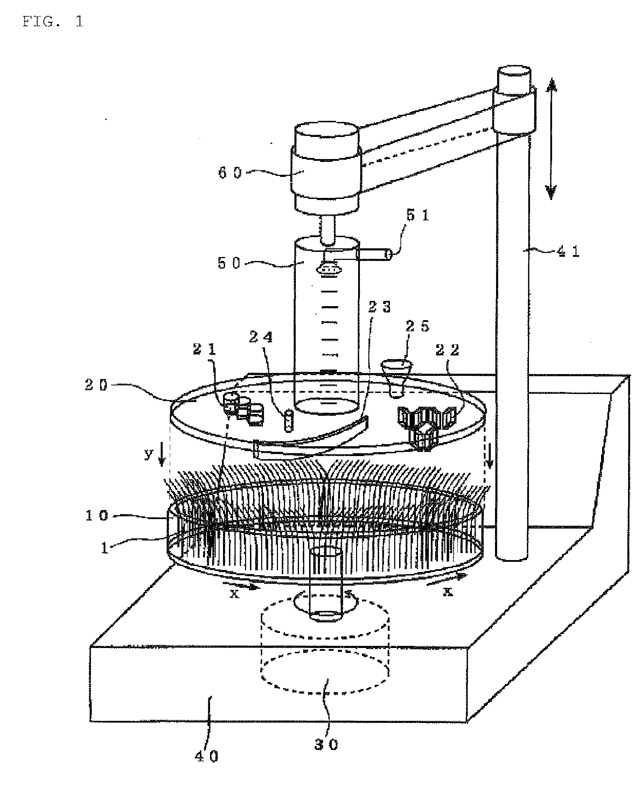

FIG. 1 is a schematic drawing illustrating a configuration of an apparatus used for evaluating quickness of foaming.

### DESCRIPTION OF THE EMBODIMENTS

The aqueous hair cleansing agent of the present invention contains the above-described components (A), (B), and water. The individual components will be explained below in detail.

First, the component (A) will be explained.

In the aqueous hair cleansing agent of the present invention, the component (A) is a sulfate-type anionic surfactant, which is exemplified by alkylsulfate or alkyl ether sulfate, represented by the general formula (1).
(A) Sulfate-Type Anionic Surfactant Represented by the Following General Formula (1):

   R¹O(CH₂CH₂O)ₙSO₃M (1)

   (in the general formula (1), R¹ represents an alkyl group or alkenyl group having 10 to 18 carbon atoms, M represents an alkali metal, alkali earth metal, ammonium, alkanolamine or basic amino acid, and n represents a number of 0 to 5 estimated based on weight average.)
   Among these, in view of ensuring quick foamability and excellent cleansing performance, polyoxyethylene alkyl ether sulfate having an alkyl group of 12 to 14 carbon atoms for R¹, 1 to 2 for n estimated based on weight average, and ammonium or sodium for M in the general formula (1), is preferable.
   The component (A) may be a single species, or may be a combination of two or more species, wherein the content of which is preferably 1 to 25% by weight, more preferably 5 to 23% by weight, and even more preferably 8 to 20% by weight of the aqueous hair cleansing agent of the present invention, in view of foamability, pH under condition of use, and finger combability during rinsing.
   Next, the component (B) will be explained.
   In the aqueous hair cleansing agent of the present invention, the component (B) is a polyoxyethylene alkyl ether-type nonionic surfactant represented by the general formula (2).
(B) Polyoxyethylene Alkyl Ether-Type Nonionic Surfactant Represented by the Following General Formula (2):

   R²O(CH₂CH₂O)ₘH (2)

   (in the general formula (2), R² represents a straight-chain or branched alkyl group or alkenyl group having 18 carbon atoms, and m represents a number of 6 to 10 estimated based on weight average.)

Representative products commercially available as the component (B) may be exemplified by polyoxyethylene alkyl ether-type surfactants such as EMALEX 606 (polyoxyethylene (6) stearyl ether, with a molecular weight of 518, from Nihon Emulsion Co., Ltd.), Emalgen 306P (polyoxyethylene (6) stearyl ether, with a molecular weight of 518, from Kao Corporation), and Emalgen 409P (polyoxyethylene (9) oleyl ether, with a molecular weight of 648, from Kao Corporation).

The nonionic surfactant which configures the component (B) in the present invention is supposed to form a protective film over the surface of the horny layer, to thereby suppress the anionic surfactant, composing the component (A), from permeating into the horny layer. In order to allow expression of this sort of effect, it is preferable that the nonionic surfactant which configures the component (B) effectively adsorb onto the surface of the horny layer, but does not permeate thereinto.

In view of allowing the component (B) to efficiently adsorb onto the surface of the horny layer, the component (B) preferably has a critical micelle concentration (CMC) of approximately 1×10⁻⁶ M or smaller. The CMC value decreases as the length of alkyl chain elongates. In view of achieving both of adsorptivity onto the horny layer and foamability of the hair cleansing agent at the same time, R² in the above-described formula (2) is preferably a polyoxyethylene stearyl ether having 18 carbon atoms.

In order to avoid permeation of the component (B) into the horny layer, the component (B) preferably has a molecular weight of 500 or larger. In the above-described formula (2), m is preferably 6 to 10. In particular, m is preferably 6. More specifically, polyoxyethylene (6) stearyl ether may be exemplified.

The component (B) may be a single species, or may be a combination of two or more species, wherein the content of which is preferably 0.1 to 10% by weight, more preferably 0.5 to 7% by weight, and even more preferably 1 to 5% by weight of the aqueous hair cleansing agent of the present invention, in view of mildness to the scalp, foamability, shortening of rinsing time, and finger combability during rinsing.

In view of achieving both mildness to the scalp and good foamability, the components (A), (B) preferably give the weight ratio of the component (B) relative to the total of the components (A) and (B) (i.e. (B)/((A)+(B))), of from 1/100 to 25/100 (1 to 25%), more preferably 3/100 to 23/100 (3 to 23%), and even more preferably 5/100 to 20/100 (5 to 20%).

In order to further improve the effect of suppressing dandruff, itchiness, rash and cracking of the scalp, the aqueous hair cleansing agent of the present invention may further contain an organic carboxylic acid and anti-inflammatory agent.

The organic carboxylic acid preferably has 2 to 6 carbon atoms, and is more preferably dicarboxylic acid (which may have a hydroxy group) and hydroxymonocarboxylic acid. More specifically, the dicarboxylic acid may be exemplified by malonic acid, succinic acid, glutaric acid, adipic acid, maleic acid, fumaric acid, phthalic acid, and oxalic acid. The dicarboxylic acid having a hydroxy group may be exemplified by malic acid and tartaric acid. The hydroxymonocarboxylic acid may be exemplified by glycolic acid, lactic acid, hydroxyacrylic acid, oxybutyric acid, glyceric acid, and gluconic acid.

Among these organic carboxylic acids, preferable examples include glycolic acid, lactic acid, malonic acid, maleic acid, and malic acid. Salts of these organic carboxylic acids may be exemplified by those formed with alkali metal, alkali earth metal, ammonia, and organic amine compound.

The organic carboxylic acid or salt thereof may be a single species, or may be a combination of two or more species, wherein the content of which is preferably 0.1 to 5% by weight, more preferably 0.2 to 3% by weight, and even more preferably 0.5 to 2% by weight of the aqueous hair cleansing agent, in view of ensuring a sufficient level of scalp improvement.

In view of maximizing the effect of improving scalp conditions by the organic carboxylic acid, the aqueous hair cleansing agent of the present invention preferably has a pH value of 1 to 5, preferably 2 to 4.5, and even more preferably 3 to 4.5, at 25°C when diluted 20-fold by weight with water.

For the purpose of adjusting the pH of the composition within such desirable range, an organic or inorganic acid, or an alkali agent may be used while being appropriately combined and quantified. The organic acid may be exemplified by citric acid and glutamic acid, besides the above-described organic carboxylic acid. The inorganic acid may be exemplified by hydrochloric acid, sulfuric acid, and phosphoric acid. The alkali may be exemplified by sodium hydroxide, and potassium hydroxide.

The aqueous hair cleansing agent of the present invention is excellent in foamability, smoothness of foam, and smooth rinsability, and expresses an effect of suppressing swelling of the horny layer, and itchiness, irrespective of the pH value. The cleansing agent may also successfully obtain an additional anti-dandruff effect which could not be obtained under a neutral condition, if the cleansing agent contains the organic carboxylic acid or salt thereof so as to adjust the pH to 1 to 5 at 25°C when diluted 20-fold by weight with water.

Examples of the anti-inflammatory agent includes glycyrrhetinic acid, glycyrrhizic acid and derivatives thereof; and also include components having anti-inflammatory function, such as *ε*-aminocaproic acid, allantoin, sodium guaiazulene sulfonate, d-camphor, *l*-menthol, urea, pyridoxine dipalmitate, glycyrrhetinyl stearate, tranexamic acid, vitamin A oil, hydrocortisone, prednisolone, pyridoxine hydrochloride, Kankohso (photosensitizer) 301, zinc oxide, and hydrocortisone acetate. The derivatives of glycyrrhetinic acid or glycyrrhizic acid include salts which include alkali metal salts represented by sodium salt and potassium salt, and ammonium salt; and esters such as glycerin ester and stearyl ester. More specifically, dipotassium glycyrrhizinate, disodium glycyrrhizinate, trisodium glycyrrhizinate, monoammonium glycyrrhizinate, glycerin glycyrrhetinate, and stearyl glycyrrhetinate may be exemplified.

The anti-inflammatory agent may be a single species, or may be a combination of two or more species, wherein the content of which is preferably 0.001 to 10% by weight, more preferably 0.005 to 5% by weight, and even more preferably 0.01 to 1% by weight of the aqueous hair cleansing agent.

From another aspect, the hair cleansing agent may occasionally be required to more effectively suppress dandruff and itchiness of the scalp. It is also generally known that killing of resident bacteria is one effective means for suppressing dandruff. The hair cleansing agent is often added also with antibacterial agent.

An exemplary hair cleansing agent which contains alkyl ether sulfate as a main cleansing component, together with the antibacterial agent, may be found in Patent Document 4. While the cleansing performance and anti-dandruff performance are reportedly good by virtue of the combination of the anionic surfactant and the antibacterial agent, the hair cleansing agent still has room for improvement as for suppression of itchiness.

From this point of view, the present invention provides an aqueous hair cleansing agent containing an alkylsulfate or alkyl ether sulfate as a main cleansing component, which is excellent in mildness to the scalp and foamability, and strongly effective in suppressing dandruff and itchiness of the scalp, by virtue of the combination of a specific polyoxyethylene alkyl ether-type nonionic surfactant with the antibacterial agent.

This sort of antibacterial agent (referred to as "component (C)", hereinafter) may be exemplified by triclosan, triclocarban, pirocton olamine, zinc pyrithione, selenium disulfide, 3-methyl-4-(1-methylethyl)phenol, benzalkonium salt or benzethonium salt represented by the general formula (3), and pyridinium salt represented by the general formula (5).

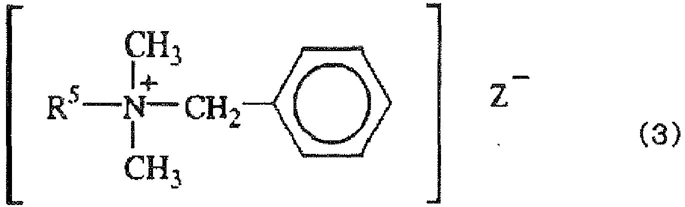

(In the formula, R⁵ represents an alkyl group having 8 to 18 carbon atoms or a group represented by formula (4), and Z⁻ represents a halogen ion.)

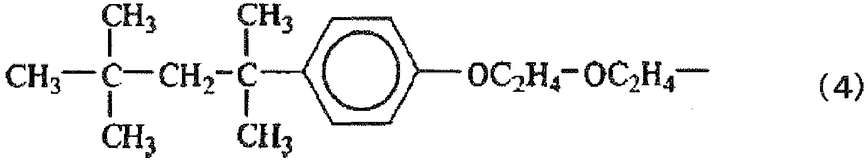

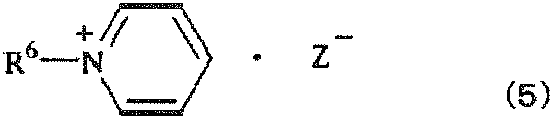

(In the formula, R⁶ represents a straight-chain or branched alkyl group having 6 to 18 carbon atoms, and Z⁻ represents a halogen ion.)

In view of obtaining a sufficient level of antibacterial performance, the component (C) is preferably mixed into the hair cleansing agent in an amount of from 0.1 to 4% by weight, particularly 0.4 to 3% by weight.

The component (C) is preferably zinc pyrithione, pirocton olamine, or benzalkonium salt having an alkyl group of 12 to 16 carbons for R⁵ in the general formula (3). Zinc pyrithione and pirocton olamine are more preferable.

While the aqueous hair cleansing agent of the present invention is excellent in foamability, smoothness of foam and smooth rinsability, suppresses swelling of the horny layer, and suppresses itchiness irrespective of the pH value, the pH value when applied to hair (diluted 20-fold with water at 25°C) is preferably 5 to 8, in view of stability of the antibacterial agent such as zinc pyrithione. The pH may be adjustable by using any pH adjusting agents (acid, alkali) generally adopted.

The aqueous hair cleansing agent of the present invention may contain an anionic surfactant other than the component (A), and a nonionic surfactant or amphoteric surfactant other than the component (B), for the purpose of further improving the cleansing performance.

The anionic surfactant other than the component (A) adoptable herein includes sulfuric acid-based ones, sulfonic acid-based ones, and carboxylic acid-based ones. The examples include sulfosuccinic acid alkylene alkylphenyl ether sulfate, sulfosuccinic acid alkyl ester salt, polyoxyalkylene sulfosuccinic acid alkyl ester salt, alkane sulfonate, higher fatty acid salts, alkyl ether carboxylic acid and salt thereof. Among these, alkyl ether carboxylic acid or salt thereof is preferable.

The nonionic surfactant other than the component (B) adoptable herein may be exemplified by polyoxyalkylene solbitan fatty acid ester, polyoxyalkylene solbit fatty acid ester, polyoxyalkylene glycerin fatty acid ester, polyoxyalkylene fatty acid ester, polyoxyalkylene alkyl ether, polyoxyalkylene alkylphenyl ether, polyoxyalkylene (hydrogenated)castor oil, sucrose fatty acid ester, polyglycerin alkyl ether, polyglycerin fatty acid ester, fatty acid alkanolamide, alkylglycoside, mono alkyl and monoalkenyl glyceryl ether.

Among these, polyoxyalkylene solbitan fatty acid ester such as polyoxyethylene solbitan fatty acid ester, polyoxyalkylene fatty acid ester such as polyoxyalkylene (C₈ to C₂₀) fatty acid ester, and polyoxyalkylene (hydrogenated) castor oil such as polyoxyethylene hydrogenated castor oil, and alkylglycoside are preferable.

Also fatty acid alkanolamide is preferable, allowing either of monoalkanolamide and dialkanolamide. Those having an acyl group of 8 to 18 carbon atoms, in particular 10 to 16 carbon atoms are preferable. Also those having a hydroxyalkyl group of 2 to 3 carbon atoms are preferable, which are exemplified by oleic acid diethanolamide, palm kernel oil fatty acid diethanolamide, coconut oil fatty acid diethanolamide, lauric acid diethanolamide, polyoxyethylene coconut oil fatty acid monoethanolamide, coconut oil fatty acid monoethanolamide, lauric acid isopropanolamide, and lauric acid monoethanolamide.

Also glyceryl monoalkyl ether or glyceryl monoalkenyl ether is preferable, where the alkyl group or alkenyl group is preferably a straight-chain or branched alkyl group having 4 to 10 carbon atoms, in particular 8 to 10 carbon atoms. The specific examples include *n*-butyl group, isobutyl group, *n*-pentyl group, 2-methylbutyl group, isopentyl group, *n*-hexyl group, isohexyl group, *n*-heptyl group, *n*-octyl group, 2-ethylhexyl group, *n*-decyl group, and isodecyl group. In particular, 2-ethylhexyl group and isodecyl group are preferable.

The amphoteric surfactant may be exemplified by betaine-based surfactants. Among these, alkyl dimethyl aminoacetic acid betaine, fatty acid amidopropyl betaine, and alkyl hydroxy sulfobetaine are preferable, wherein fatty acid amidopropyl betaine is more preferable. The fatty acid amidopropyl betaine preferably has an acyl group of 8 to 18 carbon atoms, in particular 10 to 16 carbon atoms, wherein more preferable examples include lauric acid amidopropyl betaine, palm kernel oil fatty acid amidopropyl betaine, and coconut oil fatty acid amidopropyl betaine.

These surfactants may be a single species, or may be a combination of two or more species contained in the aqueous hair cleansing agent. In view of obtaining the aqueous hair cleansing agent of the present invention in the form of a water-based liquid cleaner, it is preferable to use fatty acid amidopropyl betaine, fatty acid alkanolamide, or mono alkylglyceryl ether, together with the components (A), (B), not only because the foamability is improved, but also because the appropriate level of pH may be obtained.

The content of these surfactants is preferably 0.1 to 15% by weight in the hair cleansing agent of the present invention, in view of obtaining a good effect of enhancing the foamability. From this point of view, the content is more preferably 0.5 to 8% by weight, and even more preferably 1 to 6% by weight.

The aqueous hair cleansing agent of the present invention may further be added with a cationic surfactant, a cationized polymer or silicones, for the purpose of improving the finger combability during rinsing, and of improving styling of hair after being dried.

The cationic surfactant may be exemplified by alkyltrimethyl ammonium salt, alkoxy trimethyl ammonium salt, dialkyldimethyl ammonium salt, alkyldimethylamine salt, alkoxy dimethylamine salt, and alkylamide dimethylamine salt.

### (i) Alkyl Trimethylammonium Salt

This is exemplified by those represented by the following general formula:

R¹¹-N⁺(CH₃)₃X

(in the formula, R¹¹ represents an alkyl group having 12 to 22 carbon atoms, and X represents a halogen (chlorine or bromine) ion.)

More specifically, cetyl trimethylammonium chloride, stearyl trimethylammonium chloride, and behenyl trimethylammonium chloride may be exemplified.

### (ii) Alkoxy Trimethylammonium Salt

This is exemplified by those represented by the following general formula:

R¹²-O-R¹³-N(CH₃)₃X

(in the formula, R¹² represents an alkyl group having 12 to 22 carbon atoms, R¹³ represents an ethylene group or a propylene group, and X is same as described in the above.)

More specifically, stearoxypropyl trimethylammonium chloride, stearoxyethyl trimethylammonium chloride, and stearoxyhydroxypropyl trimethylammonium chloride may be exemplified.

### (iii) Dialkyldimethyl Ammonium Salt

This is exemplified by those represented by the following general formula:

R¹⁴₂-N⁺(CH₃)₂X

(in the formula, R¹⁴ represents an alkyl group having 12 to 22 carbon atoms or a benzyl group, and X is same as described in the above.)

More specifically, distearyl dimethyl ammonium chloride may be exemplified.

### (iv) Alkyldimethylamine Salt

This is exemplified by those represented by the following general formula:

R¹⁵-N(CH₃)₂

(in the formula, R¹⁵ represents an alkyl group having 12 to 22 carbon atoms.)

More specifically, organic acid salts of behenyl dimethylamine and stearyl dimethylamine may be exemplified.

### (v) Alkoxydimethylamine Salt

This is exemplified by those represented by the following general formula:

R¹⁶-O-R¹⁷-N(CH₃)₂

(in the formula, R¹⁶ represents an alkyl group having 12 to 22 carbon atoms, and R¹⁷ represents an ethylene group or a propylene group.)

### (vi) Alkylamide Dimethylamine Salt

This is exemplified by those represented by the following general formula:

R¹⁸-C(=O)NH-R¹⁹-N(CH₃)₂

(in the formula, R¹⁸ represents an alkyl group having 11 to 21 carbon atoms, and R¹⁹ represents an ethylene group or a propylene group.)

Cationic surfactants other than the above-described (i) to (vi) may be exemplified by lanolin fatty acid aminopropylethyldimethyl ammonium ethyl sulfate, lanolin fatty acid aminoethyltriethylammonium ethyl sulfate, lanolin fatty acid aminopropyltriethylammonium ethyl sulfate, lanolin fatty acid aminoethyltrimethylammonium methyl sulfate, lanolin fatty acid aminopropylethyldimethylammonium methyl sulfate, isoalkanoic acid (C₁₄ to C₂₀) aminopropylethyldimethylammonium ethyl sulfate, isoalkanoic acid (C₁₈ to C₂₂) aminopropylethyldimethylammonium ethyl sulfate, isostearic acid aminopropylethyldimethylammonium ethyl sulfate, isononanoic acid aminopropylethyldimethylammonium ethyl sulfate and alkyltrimethylammonium saccharin.

Two or more species of the cationic surfactant may be combined, wherein the content of which is preferably 0.01 to 10% by weight of the aqueous hair cleansing agent of the present invention, more preferably 0.05 to 5% by weight, and even more preferably 0.1 to 2% by weight, in view of smoothness over a period from washing to rinsing.

Next, the cationized polymer may be exemplified by cationized cellulose, cationized starch, cationized fenugreek gum, cationized guar gum, cationized tara gum, cationized locust bean gum, cationized xanthan gum, diallyl quaternary ammonium salt/acrylamide copolymer, vinylimidazolium trichloride/vinylpyrrolidone copolymer, hydroxyethylcellulose/dimethyldiallyl ammonium chloride copolymer, vinylpyrrolidone/quaternized dimethylaminoethyl methacrylate copolymer, polyvinylpyrrolidone/alkylamino acrylate copolymer, polyvinylpyrrolidone/alkylamino acrylate/vinylcaprolactam copolymer, vinylpyrrolidone/methacrylamidopropyl trimethylammonium chloride copolymer, alkylacrylamide/acrylate/alkylamino alkylacrylamide/polyethylene glycol methacrylate copolymer, adipic acid/dimethylaminohydroxypropylethylene triamine copolymer (Cartaretine from Sandoz US), and cationic polymers described in Japanese Patent Publication Nos. JP-A-S53-139734 and JP-A-S60-36407. In particular, cationized cellulose, cationized fenugreek gum, cationized guar gum, cationized tara gum, cationized locust bean gum, and diallyl quaternary ammonium salt/acrylamide copolymer are preferable.

The cationized polymer is also commercially available under trade names of Merquat 550 (from NALCO Company, copolymer of acrylamide and diallyl dimethyl ammonium salt; CTFA name=Polyquaternium-7), Luviquat FC370 (from BASF Corporation, copolymer of 1-vinyl-2-pyrrolidone and 1-vinyl-3-methylimidazolium salt; CTFA name=Polyquaternium-16), Gafquat 755N (from ISP, Inc., copolymer of 1-vinyl-2-pyrrolidone and dimethylaminoethyl methacrylate; CTFA name=Polyquaternium-11), Ucare polymer JR and Ucar polymer LR Series (from Amerchol Corporation, salts of reaction product between trimethylammonium-substituted epoxide and hydroxyethylcellulose; CTFA name=Polyquaternium-10), Poiz C-60H, Poiz C-80M, Poiz C-150L (from Kao Corporation, salts of reaction product between trimethylammonium-substituted epoxide and hydroxyethylcellulose; CTFA name=Polyquaternium-10), Jaguar Series (from Rhodia, salt of reaction product between trimethylammonium-substituted epoxide and guar gum), Catinal CF-100 (from TOHO Chemical Industry Co., Ltd., salt of reaction product between trimethylammonium-substituted epoxide and fenugreek gum), Catinal CTR-100 (from TOHO Chemical Industry Co., Ltd., salt of reaction product between trimethylammonium-substituted epoxide and tara gum), and Catinal CLB-100 (from TOHO Chemical Industry Co., Ltd., salt of reaction product between trimethylammonium-substituted epoxide and locust bean gum).

Two or more species of these cationized polymers may be combined, wherein the content of which is preferably 0.01 to 3% by weight of the aqueous hair cleansing agent of the present invention, more preferably 0.05 to 2% by weight, and even more preferably 0.1 to 1% by weight, in view of smoothness over a period from washing to rinsing.

The silicones may be exemplified by those listed below.

### (1) Dimethylpolysiloxane

This is exemplified by those represented by the following general formula (6):

(CH₃)₃Si-[(CH₃)₂SiO]_{d}-Si(CH₃)₃ (6)

(in the formula, d represents a number of 3 to 2×10⁴.)

The dimethylpolysiloxane exist as dispersed particles in the aqueous hair cleansing agent, wherein the average particle size of the dispersed particles is preferably 0.1 to 100 µm, more preferably 0.1 to 50 µm, even more preferably 0.1 to 4 µm, and even more preferably 0.1 to 2 µm, in view of excellence of styling after drying, and storage stability of the hair cleansing agent.

The average particle size of the polydimethylsiloxane emulsion herein means the median diameter measured by laser light scattering method, and may be measured using a general particle analyzer based on laser light scattering, such as LS-130 from Coulter Corporation.

This sort of dimethylpolysiloxane is commercially available under the name of "Silicone CF2450" from Dow Corning Toray Co., Ltd. which contains 60% by weight of dimethylpolysiloxane oil represented by the formula (6) with d=300 to 6,500, and has an average particle size of 0.8 µm; or under the name of "Silicone CF2460" from Dow Corning Toray Co., Ltd., which contains 75% by weight of dimethylpolysiloxane oil with d=300 to 6,500, and has an average particle size of 20 µm.

The dimethylpolysiloxane is preferably contained in an amount of from 0.01 to 10% by weight of the aqueous hair cleansing agent of the present invention, preferably 0.05 to 6% by weight, more preferably 0.3 to 3% by weight, and even more preferably 0.5 to 2% by weight, in view of improving foam texture, and texture or gloss of hair after being dried.

### (2) Amino-Modified Silicone

While various amino-modified silicones may be adoptable, a product listed under the name of Amodimethicone in the CTFA Dictionary (Cosmetic Ingredient Dictionary, USA), 9th Edition, 2002, Volume 1, p.107, having an average molecular weight of approximately 3000 to 100,000, is preferable. The commercially available products may be exemplified by SM 8704C (from Dow Corning Toray Co., Ltd.), DC 929 (from Dow Corning Corporation), KT 1989 (from Momentive Performance Materials Inc.), 8500 Conditioning Agent, DOW CORNING TORAY SS-3588, and DOW CORNING TORAY SILSTYLE 104 (from Dow Corning Toray Co., Ltd.).

### (3) Other Silicones

Other silicones besides those described above may be exemplified by polyether-modified silicone, methylphenyl polysiloxane, aliphatic acid-modified silicone, alcohol-modified silicone, alkoxy-modified silicone, epoxy-modified silicone, fluorine-modified silicone, cyclic silicone, and alkyl-modified silicone.

Two or more species of these amino-modified silicone and other silicones may be combined, wherein the content of which is preferably 0.01 to 5% by weight of the aqueous hair cleansing agent of the present invention, more preferably 0.05 to 2% by weight, and even more preferably 0.1 to 1% by weight, in view of smoothness over a period from washing to rinsing.

The aqueous hair cleansing agent of the present invention may further contain a pearlescent agent containing ethylene glycol monofatty acid ester, ethylene glycol difatty acid ester, ethylene glycol mono alkyl ether or ethylene glycol dialkyl ether.

The ethylene glycol monofatty acid ester may be exemplified by ethylene glycol monostearate and ethylene glycol monobehenate, and the ethylene glycol difatty acid ester may be exemplified by ethylene glycol distearate and ethylene glycol dibehenate. The ethylene glycol mono alkyl ether may be exemplified by ethylene glycol monostearyl ether, and the ethylene glycol dialkyl ether may be exemplified by ethylene glycol distearyl ether.

Each of them may be a combination of two or more species, wherein the content of which is preferably 0.1 to 10% by weight of the aqueous hair cleansing agent of the present invention, more preferably 0.5 to 5% by weight, and even more preferably 1 to 4% by weight, in view of improving storage stability, smoothness during foaming and rinsing, and stability of the hair cleansing agent.

The aqueous hair cleansing agent of the present invention may contain an oil component as a conditioning agent. The oil component may be exemplified by hydrocarbon oils such as squalene, squalane, liquid paraffin, liquid isoparaffin, and cycloparaffin;
glycerides such as castor oil, cacao oil, mink oil, avocado oil, olive oil, sunflower oil, and camellia oil; waxes such as beeswax, spermaceti, lanolin, and carnauba wax; higher alcohols such as cetyl alcohol, oleyl alcohol, stearyl alcohol, isostearyl alcohol, 2-octyl dodecanol, glycerin, myristyl alcohol, behenyl alcohol, and cetostearyl alcohol; ester oils such as isopropyl palmitate, isopropyl myristate, octyldodecyl myristate, hexyl laurate, cetyl lactate, propylene glycol monostearate, oleyl oleate, hexadecyl 2-ethylhexanoate, isononyl isononanoate, and tridecyl isononanoate;
higher fatty acids such as capric acid, lauric acid, myristic acid, palmitic acid, stearic acid, behenic acid, oleic acid, coconut oil fatty acid, isostearic acid, and isopalmitic acid; and
other materials such as isostearyl glyceryl ether, and poly(oxypropylene)butyl ether. Among these, higher fatty acids, higher alcohol, and glyceride are preferable, and lauric acid, myristyl alcohol, cetyl alcohol, stearyl alcohol, sunflower oil, and camellia oil are more preferable. These oil components may be a single species, or may be a combination of two or more species, wherein the content of which is preferably 0.1 to 2% by weight of the aqueous hair cleansing agent of the present invention, more preferably 0.2 to 1.5% by weight, and even more preferably 0.3 to 1% by weight.

The aqueous hair cleansing agent of the present invention may contain a viscosity adjusting agent. The viscosity adjusting agent may be exemplified by hydroxyethylcellulose, methylcellulose, polyethylene glycol, polypropylene glycol, ethylene glycol, propylene glycol, isoprene glycol, ethanol, benzyl alcohol, benzyloxy ethanol, phenoxy ethanol, and clay minerals, salts (sodium chloride, ammonium chloride, sodium citrate, and so forth), among which benzyl alcohol, ethanol, polypropylene glycol, sodium chloride, and sodium citrate are preferable. The viscosity adjusting agent may be a combination of two or more species, wherein the content of which is preferably 0.01 to 5% by weight of the aqueous hair cleansing agent of the present invention, more preferably 0.05 to 4% by weight, and even more preferably 0.1 to 3% by weight, in view of richness and quality of foam.

Besides the above-described components, any components adopted to general hair cleansing agent may appropriately be added to the aqueous hair cleansing agent of the present invention, depending on the need. The components may be exemplified by an antiseptic agent; chelating agent; moisturizers such as sorbitol and panthenol; colorants such as dye and pigment; extracts such as polar solvent extract of eucalyptus, protein obtained from shell having a nacreous layer or from pearl, or hydrolysate thereof, honey, royal jelly, silk-derived protein or hydrolysate thereof, protein-containing extract obtained from leguminous plant seed, *Panax ginseng* extract, rice germ extract, bladderwrack extract, aloe extract, Alpinia leaf extract, and chlorella extract; pearlescent agent such as titanium oxide; perfume; UV absorber; antioxidant; and other components listed in Encyclopedia of Shampoo Ingredients (written by Anthony L.L. Hunting, 1983, published by Micelle Press)).

While the existing form of the aqueous hair cleansing agent of the present invention may appropriately be selectable from a liquid, gel and so forth, it is preferable to use water or lower alcohol, and particularly water, as a medium.

### [Examples]

### (Examples A1 to A8 and Comparative Examples A1 to A13)

Aqueous hair cleansing agents listed in Table 1 were prepared, and evaluated by the methods described below. Results are shown in Table 1. Note that pH values herein were measured at 25°C after 20-fold dilution with water.

### Method of Evaluation A1

### (1) Quickness of Foaming

Quickness of foaming was evaluated using the apparatus described in Japanese Patent Publication No. JP-A-H10-73584, according to the method described in Example of the publication.

More specifically, FIG. 1 illustrates an apparatus for evaluating foamability, having a container 10 for housing an article to be applied with the cleansing agent (hair 1), projections 21, 22 which are brought into contact with the hair 1, a gauge 50 for measuring foam produced in the container 10, a lid 20 which covers the container 10, and guides the foam produced in the container 10 towards the gauge 50, and a motor 30 for moving the container 10 so as to bring the hair housed in the container 10 and the projections 21, 22 into sliding contact. The hair 1 was wet with an equal volume of water, 1.5 mL of each sample to be evaluated and 0.3 mL of model sebum composed of lanolin were injected through an injection port 25, and the amount of foam was measured. Quickness of foaming was evaluated by the time required for the amount of foam to reach 250 mL.

The hair 1 used herein was 90 mm long, 30 g in weight in total, and attached onto a disk of approximately 160 mm in diameter. The container 10 was a cylinder of 160 mm in diameter and 20 mm high. The lid 20 had three first cylindrical projections 21 each having a diameter of 15 mm and a height of 12 mm, and nine second projections 22 each of which being 10 mm long, 2 mm wide, and 12 mm high. Number of rotation of the container 10 driven by the motor was set to 70 resolutions per minute.

Evaluation criteria are as follows:
- ⊚:: shorter than 100 seconds;
- ○:: 100 seconds or longer, shorter than 200 seconds;
- Δ:: 200 seconds or longer, shorter than 300 seconds; and
- ×:: 300 seconds or longer.

### (2) Smoothness during Foaming

Artificial hair bundle of 25 cm long, 5.5 cm wide, and 10 g in weight was lightly rinsed with 40°C water, excessive water was removed, and 0.5 g of each hair cleansing agent was thoroughly foamed thereon for approximately 30 seconds. The smoothness of hair in this process was evaluated by sensory test according to a scale of 1 to 5. Judgment was made by five persons, and an average value was found. The average of 4.0 or larger was rated "⊚", 3.2 to 3.9 was rated "○", 2.5 to 3.1 was rated "Δ", and 2.4 or smaller was rated "×":
- 5:: very smooth;
- 4:: rather smooth;
- 3:: fair;
- 2:: not so smooth; and
- 1:: not smooth.

### (3) Smoothness during Rinsing

Artificial hair bundle of 25 cm long, 5.5 cm wide, and 10 g in weight was lightly rinsed with 40°C water, excessive water was removed, and 0.5 g of each hair cleansing agent was thoroughly foamed thereon for approximately 30 seconds. The hair bundle covered with foam was then subjected to sensory test so as to evaluate smoothness, while rinsing it with 40°C water fed at a rate of 2 L/min, according to a scale of the following 1 to 5. Judgment was made by five persons, and an average value was found. The average of 4.0 or larger was rated "⊚", 3.2 to 3.9 was rated "○", 2.5 to 3.1 was rated "Δ", and 2.4 or smaller was rated "×":
- 5:: very smooth;
- 4:: rather smooth;
- 3:: fair;
- 2:: not so smooth; and
- 1:: not smooth.

### (4) Swellability of the Horny layer

Human heel was disinfected with ethanol, the horny layer was scratched off, and then thoroughly dried to obtain a horny layer powder. Twenty milligrams of the thus-dried horny layer powder was placed in a 5-mm-diameter tube for NMR measurement, 0.8 mL of each hair cleansing agent diluted 20-fold with deionized water was injected thereinto, and the level of height of the mixture after being allowed to stand for 2 hours was measured. Evaluation was expressed by a relative value while assuming the level of height, attained when 0.8 mL of deionized water was added to 20 mg of the horny layer powder, as 100%. Larger value herein means larger level of swelling of the horny layer. Judgment criteria are as follows:
- ⊚:: less than 110%;
- ○:: 110% or more, less than 120%;
- Δ:: 120% or more, less than 125%; and
- ×:: 125% or more.

### (5) Anti-Itching Effect

Ten male subjects were asked to use each cleansing agent and to wash their hair once a day for one month. After one month, itchiness of the scalp as washed was evaluated according to a scale of 1 to 5. The average of ten subjects of 4.0 or larger was rated "⊚", 3.2 to 3.9 was rated "○", 2.5 to 3.1 was rated "Δ", and 2.4 or smaller was rated "×":
- 5:: itchiness decreased;
- 4:: itchiness slightly decreased;
- 3:: itchiness remained unchanged;
- 2:: itchiness slightly increased; and
- 1:: itchiness increased.

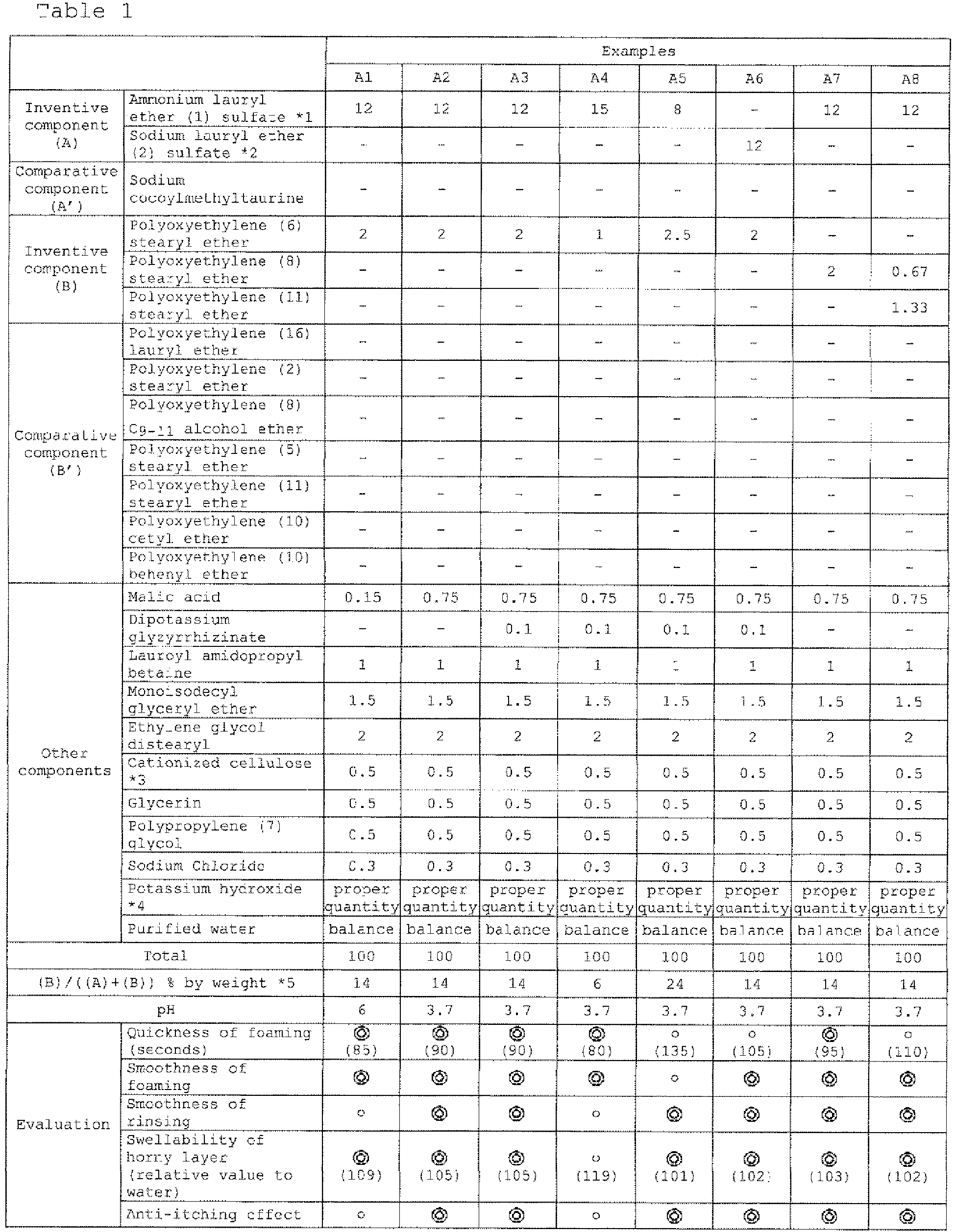

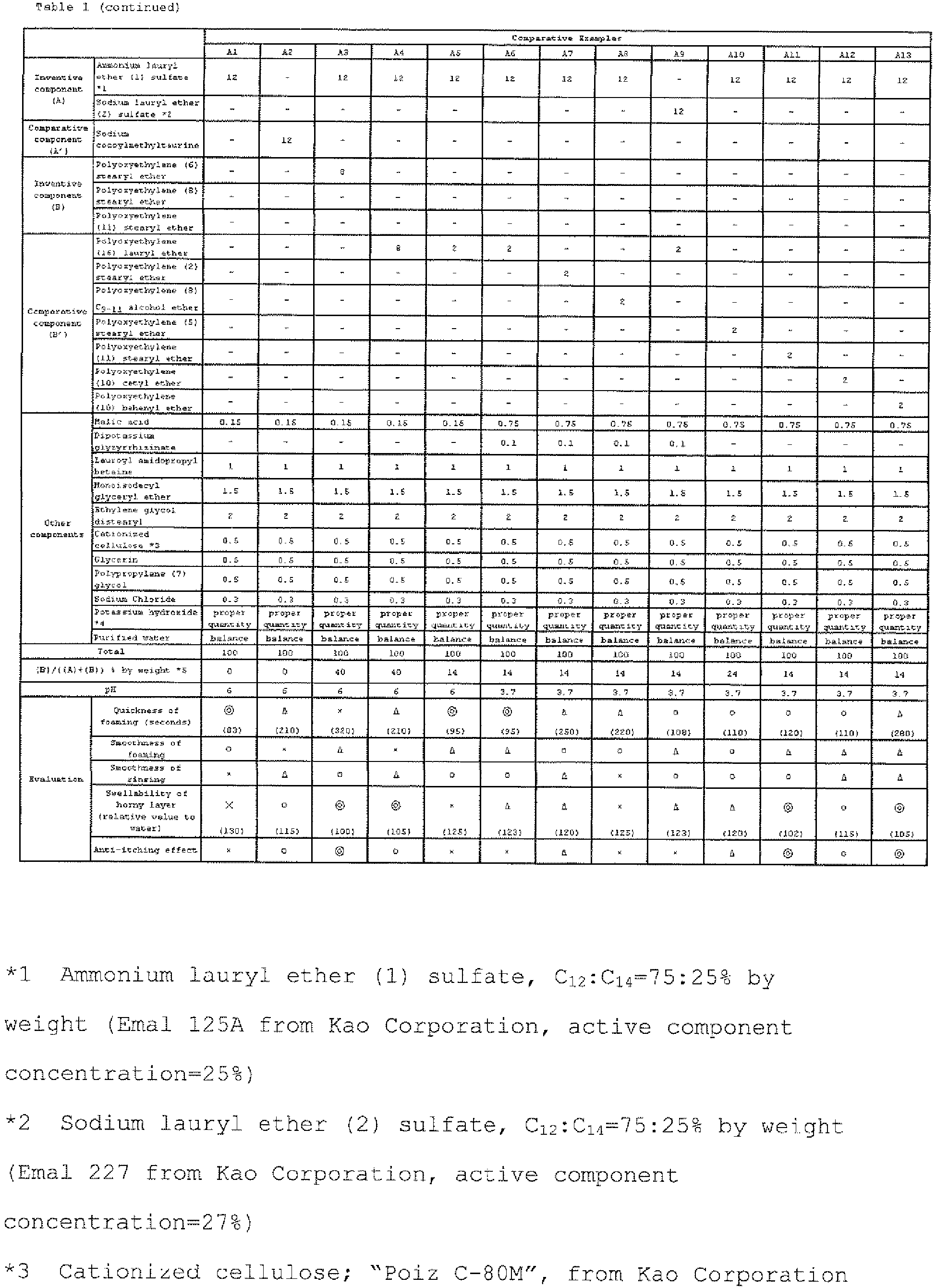

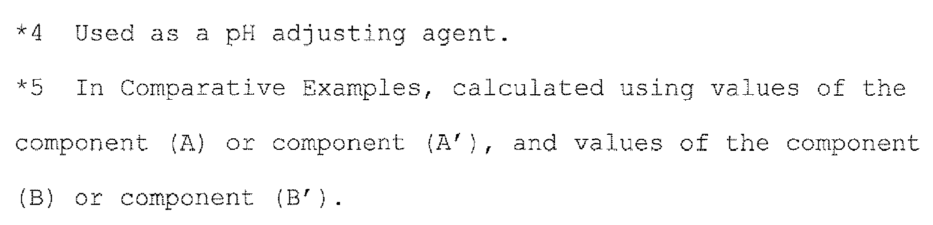

It was found from Table 1 that the compositions of Examples A1 to A8 were excellent in foamability, smoothness of foaming and smoothness of rinsing, highly effective in suppressing swelling of the horny layer, and effective in suppressing itchiness of the scalp.

### Method of Evaluation A2

Swellability of the horny layer was evaluated using formulations of Example A2 and Comparative Example A6, while varying values of "component (B) or component (B')" in "component (A) plus component (B) or component (B')" (that is, (B or B')/((A)+(B or B'))) expressed in % by weight.

Human heel was disinfected with ethanol, the horny layer was scratched off, and then thoroughly dried to obtain a horny layer powder. Twenty milligrams of the thus-dried horny layer powder was placed in a 5-mm-diameter tube for NMR measurement, 0.8 mL of each hair cleansing agent, diluted with deionized water so as to adjust the surfactant concentration to 30 mM, was injected thereinto, and the level of height of the mixture after being allowed to stand for 2 hours was measured. Evaluation was expressed by a relative value while assuming the level of height, attained when 0.8 mL of deionized water was added to 0.2 mg of the horny layer powder, as 100%. Results of the evaluation are shown below.

**Table 2**

| | (B or B')/((A)+(B or B') % by weight | | | | | | | | | | | | |
|---|---|---|---|---|---|---|---|---|---|---|---|---|---|
| | 0 | 5 | 10 | 15 | 25 | 30 | 40 | 50 | 60 | 70 | 80 | 90 | 100 |
| Example A2 | 130 | 109 | 106 | 103 | 102 | 102 | 101 | 102 | 100 | 101 | 101 | 101 | 100 |
| Comparative Example A6 | 130 | 127 | 124 | 120 | 115 | 110 | 108 | 103 | 101 | 102 | 100 | 100 | 101 |

Table 2 shows that the aqueous hair cleansing agent formulated according to Example A2 was more suppressive against swelling of the horny layer, even with a smaller amount of use of polyoxyethylene alkyl ether-type nonionic surfactant which corresponds to the component (B), as compared with the formulation according to Comparative Example A6. Since it has previously been known from the results listed in Table 1 that smaller amount of the component (B) resulted in higher level of quickness of foaming, so that, by using a specific polyoxyethylene alkyl ether-type nonionic surfactant such as that used in Examples, it is now possible to obtain an aqueous hair cleansing agent excellent in mildness to the scalp by virtue of its suppressive effect against swelling of the horny layer, and excellent in foamability by virtue of reduced amount of the component (B).

### (Examples A9 to A14)

The hair cleansing agents listed in Table 3 were prepared, and evaluated by the methods of evaluation described below. Results are shown in Table 3. Note that the pH values are measured at 25°C after 20-fold dilution with water.

### Methods of Evaluation

(1) Quickness of foaming, (2) smoothness during foaming, (3) smoothness during rinsing, (4) swellability of the horny layer, and (5) anti-itching effect were evaluated similarly as described in Examples A1 to A8 and Comparative Examples A1 to A13 (Table 1). (6) Anti-dandruff performance was evaluated according to the method described below.

### (6) Anti-Dandruff Performance

Ten male subjects were asked to wash their hair with each cleansing agent once a day for one month, then to stop washing for two days after the last washing. Hair of each subject was then washed twice using 3 g each of the same cleansing agent, and the whole volume of washate from the washing repeated twice was collected. The whole volume of collected washate was filtered through a 50 nylon mesh so as to remove unnecessary dust and hair. The whole volume of filtrate was filtered through a 255 nylon mesh (100×100 µm) preliminarily weighed, the nylon mesh was allowed to dry for approximately 48 hours at room temperature, and the amount of increase of weight thereof was determined as the weight of dandruff.

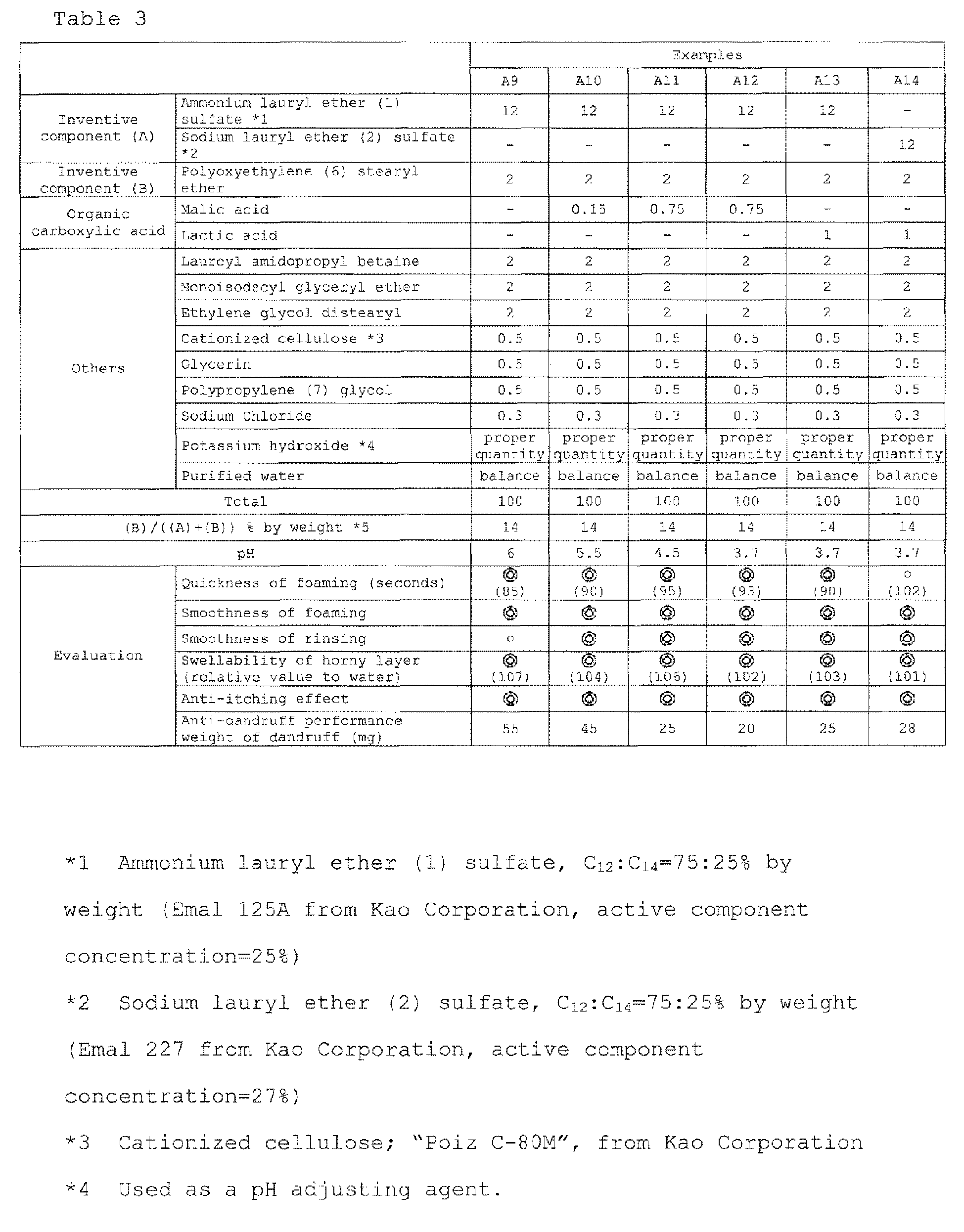

While Table 3 taught that the compositions of Examples A9 to A14 were excellent in foamability, smoothness of foam and smooth rinsability, and also showed effects of suppressing swelling of the horny layer and itchiness of the scalp, irrespective of the presence or absence of organic carboxylic acid and pH, it was also confirmed that anti-dandruff effect was additionally obtained by mixing the organic carboxylic acid so as to adjust the pH to 5 or lower.

Exemplary formulations using the aqueous hair cleansing agents of the present invention are shown below.

### (Example A15) Shampoo

| | (% by weight) |
|---|---|
| Sodium polyoxyethylene (2) lauryl ether sulfate | 12.5 |
| Polyoxyethylene (6) stearyl ether | 2.5 |
| Lactic acid | 1.0 |
| Dipotassium glycyrrhizinate | 0.1 |
| Isodecyl glyceryl ether | 2.0 |
| Laurylhydroxy sulfobetaine | 1.0 |
| Cationized guar gum | 0.2 |
| ("Jaguar C-13", from Rhodia) | |
| Cationized tara gum | 0.2 |
| ("Catinal CTR-100", from TOHO Chemical Industry Co., Ltd.) | |
| Diallyl dimethyl ammonium chloride/acrylamide copolymer | 1.2 |
| ("Merquat 550", from ONDEO Nalco Company, effective component=8.5% by weight) | |
| Dimethylpolysiloxane | 2.0 |
| ("Silicone CF2450", from Dow Corning Toray Co., Ltd., effective component=60% by weight) | |
| Ethylene glycol distearate | 2.0 |
| Myristyl alcohol | 0.2 |
| Lauric acid | 0.1 |
| Polyoxyethylene (16) lauryl ether | 0.3 |
| Polypropylene glycol (weight average molecular weight=400) | |
| | 0.5 |
| Benzyl alcohol | 0.3 |
| Ethanol | 3.0 |
| Camellia oil | 0.01 |
| Panthenol | 0.05 |
| Royal jelly | 0.01 |
| Purified honey | 0.01 |
| Silk extract | 0.05 |
| Sodium chloride | 0.2 |
| Perfume | proper quantity |
| pH adjusting agent (potassium hydroxide) | amount necessary for adjusting pH to 3.9 |
| Deionized water | balance |

### (Example A16) Shampoo

| | (% by weight) |
|---|---|
| Ammonium oxyethylene (1) lauryl ether sulfaate | 12.0 |
| Polyoxyethylene (6) stearyl ether | 2.0 |
| Malic acid | 0.75 |
| Potassium glycyrrhizinate | 0.1 |
| Isodecyl glyceryl ether | 0.8 |
| Laurylhydroxy sulfobetaine | 1.7 |
| Cationized guar gum | 0.4 |
| ("Jaguar C-13S", from Rhodia) | |
| Diallyl dimethyl ammonium chloride/acrylamide copolymer | 1.9 |
| ("Merquat 550", from ONDEO Nalco Company, effective component=8.5% by weight) | |
| Dimethylpolysiloxane | 0.5 |
| ("Silicone CF2450", Dow Corning Toray Silicone Co., Ltd., effective component=60% by weight) | |
| Ethylene glycol distearyl ester | 2.0 |
| Lauric acid | 0.8 |
| Polypropylene glycol (weight average molecular weight=1000) | 1.0 |
| Benzyl alcohol | 0.3 |
| Ethanol | 3.0 |
| Purified honey | 0.01 |
| Shea butter | 0.01 |
| Glycylglycine | 0.05 |
| Perfume | proper quantity |
| pH adjusting agent (potassium hydroxide) | amount necessary for adjusting pH to 3.9 |
| Deionized water | balance |

The hair cleansing agents of Examples A15, A16 were found to show quick foamability, combability during foaming and rinsing, and also found to be suppressive against swelling of the horny layer, mild to the skin, and suppressive against itching of the scalp. They were also found to be excellent in the anti-dandruff effect.

### (Examples B1 to B5 and Comparative Examples B1 to B8)

The hair cleansing agents listed in Table 4 were prepared, and evaluated by the methods of evaluation described below. Results are shown in Table 4. Note that pH values were measured at 25°C after 20-fold dilution with water.

### Method of Evaluation B1

### (1) Quickness of Foaming

The quickness of foaming was evaluated according to the method of evaluation described in "(1) Quickness of Foaming" of Method of Evaluation A1.

### (2) Anti-Dandruff Performance

Ten male subjects were asked to wash their hair with each cleansing agent once a day for one month, then to stop washing for two days after the last washing. Hair of each subject was then washed twice using 3 g each of the same cleansing agent, and the whole volume of washate from the washing repeated twice was collected. The whole volume of collected washate was filtered through a 50 nylon mesh so as to remove unnecessary dust and hair. The whole volume of filtrate was filtered through a 255 nylon mesh (100×100 pm) preliminarily weighed, the nylon mesh was allowed to dry for approximately 48 hours at room temperature, and the amount of increase of weight thereof was determined as the weight of dandruff. Average values of weight of dandruff from ten subjects were determined, and evaluated according to the criteria below:
- ⊚:: weight of dandruff≤30 mg;
- ○:: 30 mg<weight of dandruff≤40 mg;
- Δ:: 40 mg<weight of dandruff≤50 mg; and
- ×:: 50 mg<weight of dandruff.

### (3) Swellability of the Horny layer

Human heel was disinfected with ethanol, the horny layer was scratched off, and then thoroughly dried to obtain a horny layer powder. Twenty milligrams of the thus-dried horny layer powder was placed in a 5-mm-diameter tube for NMR measurement, 0.8 mL of each hair cleansing agent diluted 20-fold with deionized water was injected thereinto, and the level of height of the mixture after being allowed to stand for 2 hours was measured. Evaluation was expressed by a relative value while assuming the level of height, attained when 0.8 mL of deionized water was added to 20 mg of the horny layer powder, as 100%. Larger value herein means larger level of swelling of the horny layer. Judgment criteria are as follows:
- ⊚:: less than 110%;
- ○:: 110% or more, less than 120%;
- Δ:: 120% or more, less than 125%; and
- ×:: 125% or more.

### (4) Anti-Itching Effect

Ten male subjects were asked to use each cleansing agent and to wash their hair once a day for one month. After one month, itchiness of the scalp as washed was evaluated according to a scale of 1 to 5. The average of ten subjects of 4.0 or larger was rated "⊚", 3.2 to 3.9 was rated "○", 2.5 to 3.1 was rated "Δ", and 2.4 or smaller was rated "×":
- 5:: itchiness decreased;
- 4:: itchiness slightly decreased;
- 3:: itchiness remained unchanged;
- 2:: itchiness slightly increased; and
- 1:: itchiness increased.

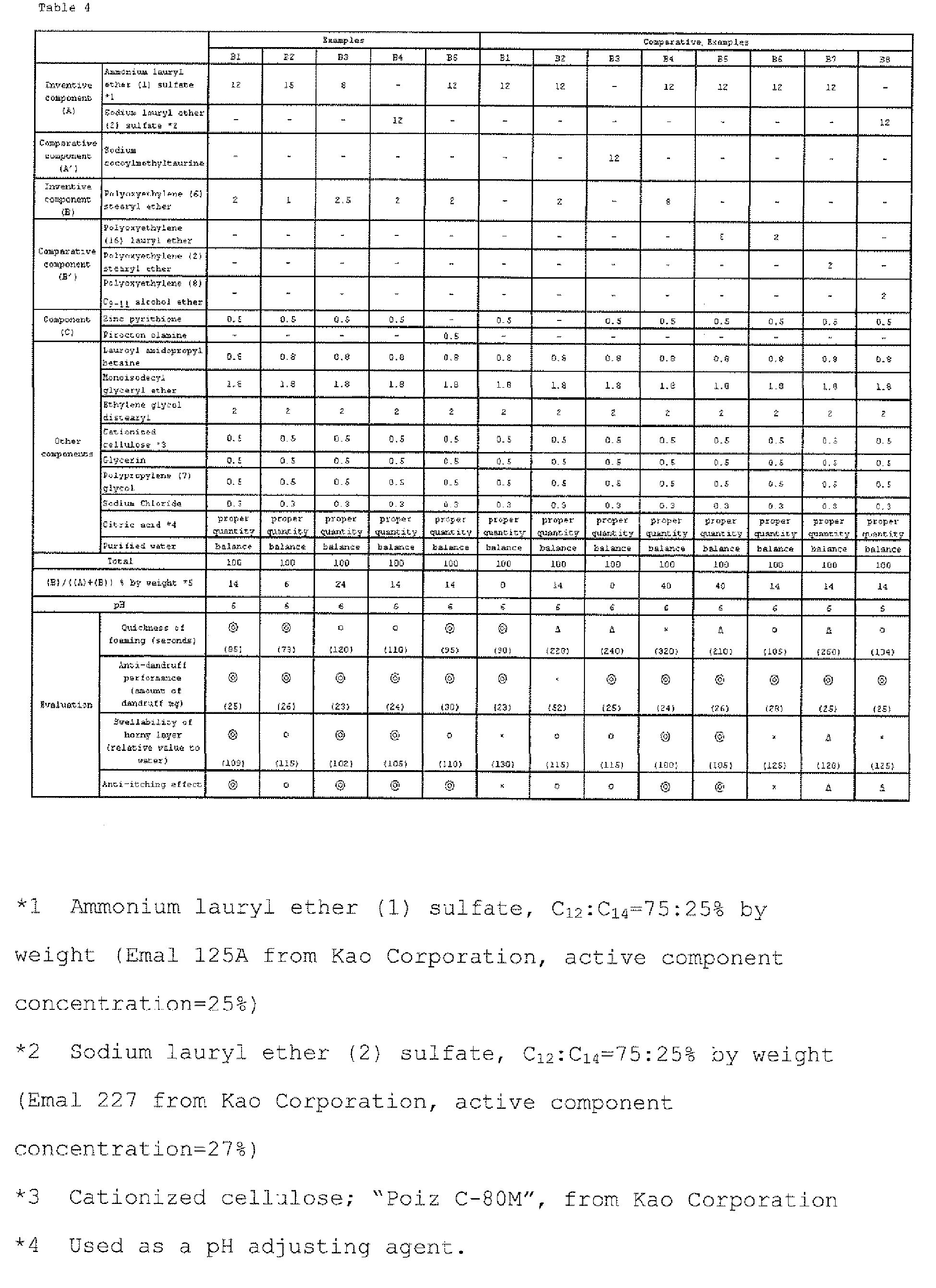

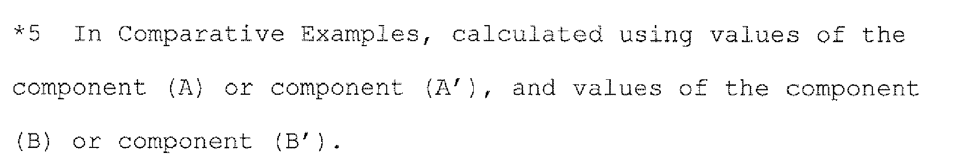

It was found from Table 4 that the compositions of Examples B1 to B5 were excellent in foamability and anti-dandruff performance, also highly effective in suppressing swelling of the horny layer, and effective in suppressing itchiness of the scalp.

### Method of Evaluation B2

Swellability of the horny layer was evaluated using formulations of Example B1 and Comparative Example B6, while varying values of "component (B) or component (B')" in "component (A) plus component (B) or component (B')" (that is, (B or B')/((A)+(B or B'))) expressed in % by weight.

Human heel was disinfected with ethanol, the horny layer was scratched off, and then thoroughly dried to obtain a horny layer powder. Twenty milligrams of the thus-dried horny layer powder was placed in a 5-mm-diameter tube for NMR measurement, 0.8 mL of each hair cleansing agent, diluted with deionized water so as to adjust the surfactant concentration to 30 mM, was injected thereinto, and the level of height of the mixture after being allowed to stand for 2 hours was measured. Evaluation was expressed by a relative value while assuming the level of height, attained when 0.8 mL of deionized water was added to 20 mg of the horny layer powder, as 100%. Results of the evaluation are shown below.

**Table 5**

| | (B or B')/((A)+(B or B')% by weight | | | | | | | | | | | | |
|---|---|---|---|---|---|---|---|---|---|---|---|---|---|
| | 0 | 5 | 10 | 15 | 25 | 30 | 40 | 50 | 60 | 70 | 80 | 90 | 100 |
| Example B1 | 130 | 112 | 106 | 103 | 102 | 102 | 101 | 102 | 100 | 101 | 101 | 101 | 100 |
| Comparative Example B6 | 130 | 127 | 124 | 120 | 115 | 110 | 105 | 103 | 101 | 102 | 100 | 100 | 101 |

Table 5 shows that the aqueous hair cleansing agent formulated according to Example B1 was more suppressive against swelling of the horny layer, even with a smaller amount of use of polyoxyethylene alkyl ether-type nonionic surfactant which corresponds to the component (B), as compared with the formulation according to Comparative Example B6. Since it has previously been known from the results listed in Table 4 that a smaller amount of the component (B) resulted in a higher level of quickness of foaming, so that, by using a specific polyoxyethylene alkyl ether-type nonionic surfactant such as that used in Examples, it is now possible to obtain an aqueous hair cleansing agent excellent in mildness to the scalp by virtue of its suppressive effect against swelling of the horny layer, and excellent in foamability by virtue of the reduced amount of the component (B).

An exemplary formulation using the aqueous hair cleansing agents of the present invention is shown below. (Example B6) Shampoo

| | (% by weight) |
|---|---|
| Sodium polyoxyethylene (2) lauryl ether sulfate | 12.5 |
| Polyoxyethylene (6) stearyl ether | 2.5 |
| Zinc pyrithione | 0.3 |
| Benzalkonium chloride (C12/C14=50/50) | 0.2 |
| Isodecyl glyceryl ether | 2.0 |
| Laurylhydroxy sulfobetaine | 1.0 |
| Cationized guar gum | 0.2 |
| ("Jaguar C-13", from Rhodia) | |
| Cationized tara gum | 0.2 |
| ("Catinal CTR-100", from TOHO Chemical Industry Co., Ltd.) | |
| Diallyl dimethyl ammonium chloride/acrylamide copolymer | 1.2 |
| ("Merquat 550", from ONDEO Nalco Company, component=8.5% by weight) | effective |
| Dimethylpolysiloxane | 2.0 |
| ("Silicone CF2450", from Dow Corning Toray Co., Ltd., effective component=60% by weight) | |
| Ethylene glycol distearate | 2.0 |
| Cetyl alcohol | 0.2 |
| Lauric acid | 0.1 |
| Polyoxyethylene (16) lauryl ether | 0.3 |
| Polypropylene glycol (weight average molecular weight=400) | 0.5 |
| Benzyl alcohol | 0.3 |
| Ethanol | 3.0 |
| Camellia oil | 0.01 |
| Panthenol | 0.05 |
| Royal jelly | 0.01 |
| Purified honey | 0.01 |
| Silk extract | 0.05 |
| Sodium chloride | 0.2 |
| Perfume | proper quantity |
| pH adjusting agent (citric acid) | amount necessary for adjusting pH to 6.5 |
| Deionized water | balance |

The hair cleansing agent of Example B6 was found to show excellent foamability and anti-dandruff performance, and also found to be suppressive against swelling of the horny layer, and suppressive against itching of the scalp.

## Claims

1. An aqueous hair cleansing agent comprising the following components (A), (B):
(A) a sulfate-type anionic surfactant represented by the following general formula (1):
R¹O(CH₂CH₂O)ₙSO₃M (1)
wherein, in the general formula (1), R¹ represents an alkyl group or alkenyl group having 10 to 18 carbon atoms, M represents an alkali metal, alkali earth metal, ammonium, alkanolamine or basic amino acid, and n represents a number of 0 to 5 estimated based on weight average; and
(B) polyoxyethylene alkyl ether-type nonionic surfactant represented by the following structural formula (2):
R²O(CH₂CH₂O)ₘH (2)
wherein, in the general formula (2), R² represents a straight-chain or branched alkyl group or alkenyl group having 18 carbon atoms, and m represents a number of 6 to 10 estimated based on weight average,
and further comprising water,
wherein the ratio of the component (B), relative to the total of the components (A) and (B) is 1/100 to 25/100 based on the weight ratio.

2. The aqueous hair cleansing agent according to Claim 1, further comprising one or more species of organic carboxylic acid or salt thereof, and having a pH of 1 to 5 at 25°C when diluted 20-fold with water.

3. The aqueous hair cleansing agent according to any one of Claim 1 or 2, further comprising at least one species of an anti-inflammatory agent in an amount of from 0.001 to 10% by weight.

4. The aqueous hair cleansing agent according to Claim 1, further comprising:
from 0.1 to 4% by weight of (C) an antibacterial agent selected from triclosan, triclocarban, pirocton olamine, zinc pyrithione, selenium disulfide, 3-methyl-4-(1-methylethyl)phenol, benzalkonium salt or benzethonium salt represented by general formula (3), and pyridinium salt represented by general formula (5): 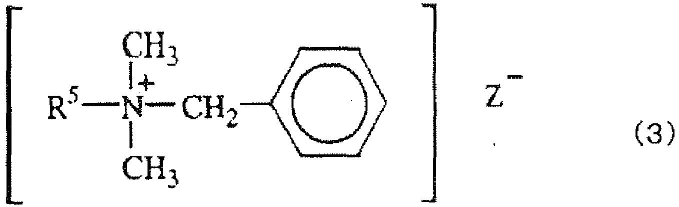 wherein, in the formula (3), R⁵ represents an alkyl group having 8 to 18 carbon atoms or a group represented by formula (4), and Z⁻ represents a halogen ion, 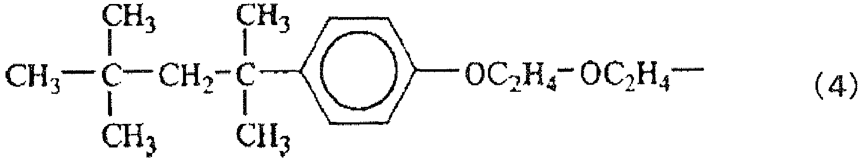 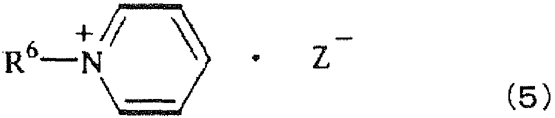 wherein, in the formula (5), R⁶ represents a straight-chain or branched alkyl group having 6 to 18 carbon atoms, and Z⁻ represents a halogen ion.

5. The aqueous hair cleansing agent according to any one of Claims 1 to 4, wherein the content of the component (A) is from 1 to 25% by weight of the aqueous hair cleansing agent.

6. The aqueous hair cleansing agent according to any one of Claims 1 to 5, wherein the content of the component (B) is from 0.1 to 10% by weight of the aqueous hair cleansing agent.

7. The aqueous hair cleansing agent according to Claim 2, wherein the content of the organic carboxylic acid or salt thereof is from 0.1 to 5% by weight of the aqueous hair cleansing agent.

8. The aqueous hair cleansing agent according to Claim 2 or 7, wherein the organic carboxylic acids is any one or two or more selected from the group consisting of glycolic acid, lactic acid, malonic acid, maleic acid, and malic acid.

9. Use of the aqueous hair cleansing agent according to any one of Claims 1 to 8 as a hair cleansing agent.

10. Use of the aqueous hair cleansing agent according to any one of Claims 1 to 8 for suppressing swelling of a horny layer of a scalp.

## Patentansprüche

1. Wässriges Haarreinigungsmittel, das die nachstehenden Komponenten (A) und (B):
(A) ein anionisches Tensid vom Sulfat-Typ der nachstehenden allgemeinen Formel (1):
R¹O(CH₂CH₂O)ₙSO₃M (1)
wobei in der allgemeinen Formel (1) R¹ eine Alkylgruppe oder Alkenylgruppe mit 10 bis 18 Kohlenstoffatomen darstellt, M ein Alkalimetall, Erdalkalimetall, Ammonium, Alkanolamin oder eine basische Aminosäure darstellt und n eine Zahl von 0 bis 5, geschätzt auf Basis des Gewichtsmittels, darstellt; und
(B) ein nicht-ionisches Tensid vom Polyoxyethylenalkylether-Typ der nachstehenden Strukturformel (2):
R²O(CH₂CH₂O)ₘH (2)
wobei in der allgemeinen Formel (2) R² eine geradkettige oder verzweigte Alkylgruppe oder Alkenylgruppe mit 18 Kohlenstoffatomen darstellt, und m eine Zahl von 6 bis 10, geschätzt auf Basis des Gewichtsmittels, darstellt,
und ferner Wasser umfasst,
wobei das Verhältnis der Komponente (B) in bezug auf die gesamten Komponenten (A) und (B) 1/100 bis 25/100 auf Basis des Gewichtsverhältnisses beträgt.

2. Wässriges Haarreinigungsmittel gemäss Anspruch 1, das ferner eine oder mehrere Art(en) einer organischen Carbonsäure oder eines Salzes davon umfasst und einen pH-Wert von 1 bis 5 bei 25°C aufweist, wenn es 20-fach mit Wasser verdünnt wird.

3. Wässriges Haarreinigungsmittel gemäss irgendeinem der Ansprüche 1 oder 2, das ferner zumindest eine Art eines entzündungshemmenden Mittels in einer Menge von 0,001 bis 10 Gew.% umfasst.

4. Wässriges Haarreinigungsmittel gemäss Anspruch 1, das ferner umfasst:
0,1 bis 4 Gew.% (C) eines Antibakteriums, ausgewählt aus Triclosan, Triclocarban, Pirocton olamin, Zinkpyrithion, Selendisulfid, 3-Methyl-4-(1-methylethyl)phenol, Benzalkoniumsalz oder Benzethoniumsalz der allgemeinen Formel (3) und Pyridiniumsalz der allgemeinen Formel (5) : 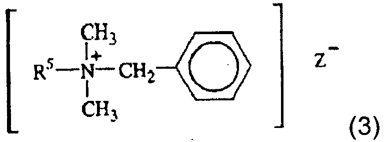 wobei in der Formel (3) R⁵ eine Alkylgruppe mit 8 bis 18 Kohlenstoffatomen oder eine Gruppe der Formel (4) darstellt und Z- ein Halogenion darstellt: 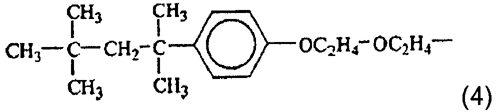 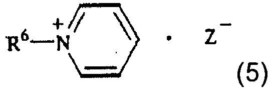 wobei in Formel (5) R⁶ eine geradkettige oder verzweigte Alkylgruppe mit 6 bis 18 Kohlenstoffatomen darstellt und Z⁻ ein Halogenion darstellt.

5. Wässriges Haarreinigungsmittel gemäss irgendeinem der Ansprüche 1 bis 4, wobei der Gehalt an Komponente (A) 1 bis 25 Gew.% des wässrigen Haarreinigungsmittels beträgt.

6. Wässriges Haarreinigungsmittel gemäss irgendeinem der Ansprüche 1 bis 5, wobei der Gehalt an Komponente (B) 0,1 bis 10 Gew.% des wässrigen Haarreinigungsmittels beträgt.

7. Wässriges Haarreinigungsmittel gemäss Anspruch 2, wobei der Gehalt an organischer Carbonsäure oder eines Salzes davon 0,1 bis 5 Gew.% des wässrigen Haarreinigungsmittels beträgt.

8. Wässriges Haarreinigungsmittel gemäss Anspruch 2 oder 7, wobei die organische Carbonsäure irgendeine oder zwei oder mehrere, ausgewählt aus der Gruppe bestehend aus Glykolsäure, Milchsäure, Malonsäure, Maleinsäure und Äpfelsäure, ist.

9. Verwendung eines wässrigen Haarreinigungsmittels gemäss irgendeinem der Ansprüche 1 bis 8 als Haarreinigungsmittel.

10. Verwendung eines wässrigen Haarreinigungsmittels gemäss irgendeinem der Ansprüche 1 bis 8 zum Unterdrücken des Anschwellens einer Hornhautschicht der Kopfhaut.

## Revendications

1. Agent aqueux de nettoyage des cheveux comprenant les composants (A), (B) suivants :
(A) un tensioactif anionique de type sulfate représenté par la formule générale (1) suivante :
R¹O(CH₂CH₂O)ₙSO₃M (1)
dans lequel, dans la formule générale (1), R¹ représente un groupe alkyle ou un groupe alcényle ayant 10 à 18 atomes de carbone, M représente un métal alcalin, un métal alcalinoterreux, l'ammonium, l'alcanolamine ou un acide aminé basique, et n représente un nombre de 0 à 5 estimé sur la base d'une moyenne de poids ; et
(B) un tensioactif non ionique de type polyoxyéthylène alkyl-éther représenté par la formule développée (2) suivante :
R²O(CH₂CH₂O)ₘH (2)
dans lequel, dans la formule générale (2), R² représente un groupe alkyle ou un groupe alcényle à chaîne linéaire ou ramifié ayant 18 atomes de carbone, et m représente un nombre de 6 à 10 estimé sur la base d'une moyenne de poids,
et comprenant en outre de l'eau,
dans lequel le rapport du composant (B) par rapport au total des composants (A) et (B) est de 1/100 à 25/100 sur la base du rapport en poids.

2. Agent aqueux de nettoyage des cheveux selon la revendication 1, comprenant en outre une ou plusieurs espèces d'acide carboxylique organique ou un sel de celui-ci, et ayant un pH de 1 à 5 à 25°C lorsqu'il est dilué avec de l'eau selon un facteur 20.

3. Agent aqueux de nettoyage des cheveux selon l'une quelconque des revendications 1 ou 2, comprenant en outre au moins une espèce d'un agent anti-inflammatoire en une quantité de 0,001 à 10 % en poids.

4. Agent aqueux de nettoyage des cheveux selon la revendication 1, comprenant en outre :
de 0,1 à 4 % en poids de (C), un agent antibactérien choisi parmi le triclosan, le triclocarban, la piroctone olamine, le pyrithione de zinc, le disulfure de sélénium, le 3-méthyl-4-(1-méthyléthyl)phénol, un sel de benzalkonium ou un sel de benzéthonium représenté par la formule générale (3), et un sel de pyridinium représenté par la formule générale (5) : 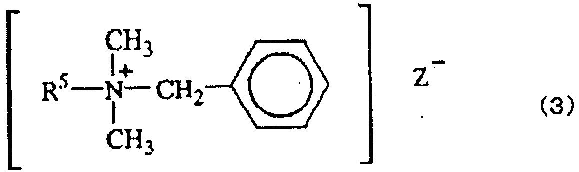 dans lequel, dans la formule (3), R⁵ représente un groupe alkyle ayant 8 à 18 atomes de carbone ou un groupe représenté par la formule (4), et Z⁻ représente un ion halogène, 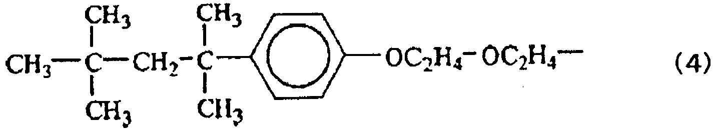 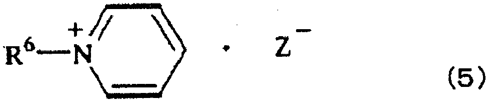 dans lequel, dans la formule (5), R⁶ représente un groupe alkyle à chaîne linéaire ou ramifié ayant 6 à 18 atomes de carbone, et Z⁻ représente un ion halogène.

5. Agent aqueux de nettoyage des cheveux selon l'une quelconque des revendications 1 à 4, dans lequel la teneur en composant (A) est de 1 à 25 % en poids de l'agent aqueux de nettoyage des cheveux.

6. Agent aqueux de nettoyage des cheveux selon l'une quelconque des revendications 1 à 5, dans lequel la teneur en composant (B) est de 0,1 à 10 % en poids de l'agent aqueux de nettoyage des cheveux.

7. Agent aqueux de nettoyage des cheveux selon la revendication 2, dans lequel la teneur en acide carboxylique organique ou d'un sel de celui-ci est de 0,1 à 5 % en poids de l'agent aqueux de nettoyage des cheveux.

8. Agent aqueux de nettoyage des cheveux selon la revendication 2 ou 7, dans lequel l'acide carboxylique organique est l'un quelconque ou deux ou plus choisis dans le groupe constitué de l'acide glycolique, l'acide lactique, l'acide malonique, l'acide maléique, et l'acide malique.

9. Utilisation de l'agent aqueux de nettoyage des cheveux selon l'une quelconque des revendications 1 à 8 comme agent de nettoyage des cheveux.

10. Utilisation de l'agent aqueux de nettoyage des cheveux selon l'une quelconque des revendications 1 à 8 pour supprimer le gonflement d'une couche cornée d'un cuir chevelu.
